(19) Europäisches Patentamt
European Patent Office
Office européen des brevets

(11) **EP 3 338 749 A1**

(12) **EUROPÄISCHE PATENTANMELDUNG**

(43) Veröffentlichungstag:
**27.06.2018  Patentblatt 2018/26**

(21) Anmeldenummer: **17002000.2**

(22) Anmeldetag: **08.12.2017**

(51) Int Cl.:
*A61F 13/511* (2006.01)    *A61F 13/512* (2006.01)
*B32B 5/02* (2006.01)    *B32B 5/26* (2006.01)
*D04H 1/495* (2012.01)    *D04H 1/498* (2012.01)
*D04H 13/00* (2006.01)

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**MA MD TN**

(30) Priorität: **23.12.2016  DE 102016015445**

(71) Anmelder: **Sandler AG**
**95126 Schwarzenbach/Saale (DE)**

(72) Erfinder:
• **Die Erfinder haben auf ihr Recht verzichtet, als solche bekannt gemacht zu werden.**

(54) **VLIESSTOFFLAMINAT**

(57)    Die Erfindung betrifft ein Vliesstofflaminat mit Struktur zur verbesserten Aufnahme und Verteilung von Körperflüssigkeiten in Hygieneprodukten. Die Aufnahme- und Verteilwirkung wird dabei durch mustermäßig verteilte Perforationen in Verbindung mit auf einer Seite des Vliesstofflaminats vorhandenen Erhöhungen bewirkt.

Fig. 1

**Beschreibung**

Wegwerfartikel für Hygieneanwendungen wie z.B. Damenbinden, Windeln sowie Inkontinenzprodukte haben den Zweck Körperausscheidungen so schnell wie möglich von der Haut wegzuleiten, um Hautirritationen zu vermeiden und dem Anwender ein sicheres und komfortables Gefühl zu geben. Für diesen Zweck sind Hygieneprodukte im Allgemeinen wie folgt aufgebaut:

- Erste Lage: Topsheet (Lochfolie oder Vlies)
- Zweite Lage: Acquisition and Distribution Layer, "ADL" (zumeist aus Vlies)
- Dritte Lage: Saugkern (bestehend aus Zellstoff und/oder Polyacrylsäure fixiert in einer oder mehreren Fixierlagen (SMS, ..)
- Abschlusslage: Folie, um ein Austreten der Flüssigkeit auf der Rückseite zu unterbinden

Das Ziel von körperkontaktierten Hygieneprodukten ist es, Körperflüssigkeiten möglichst schnell und rückstandslos aufzunehmen und an ein absorbierendes Material zu binden, das die Flüssigkeit immobilisiert. Dabei soll insbesondere vermieden werden, dass dauerhafter Körperkontakt zu den Flüssigkeiten bestehen bleibt und dass die Flüssigkeiten vor der Immobilisierung in unkontrollierter Weise aus dem Hygieneartikel austreten und die Unterwäsche verschmutzen oder gar als sichtbare Flecken auf der Oberbekleidung erscheinen.

Um dieses Ziel zu erreichen wurden im Allgemeinen Mehrlagenverbunde, bestehend aus einem Liner (Topsheet), einer Flüssigkeitsaufnahme- und Verteilschicht, einem absorbierenden Kern und einer flüssigkeitsundurchlässigen Folie als Wäscheschutz eingesetzt. Diese, in der Regel als Einzellagen hergestellten Vliesstoffe und Folien werden thermisch, chemisch oder mittels Ultraschall zu der Funktionseinheit verbunden.

Es hat sich jedoch gezeigt, dass diese herkömmlichen Verbunde nur niedrigviskose, homogene wässrige Flüssigkeiten mit newtonschem Fließverhalten, wie Urin gut aufzunehmen und zu binden vermögen. Grund hierfür ist der überwiegend kapillare Transportmechanismus, der auf der feinporigen Struktur der verwendeten Vliesstoffe basiert.

Kapillarität wirkt sich aber kontraproduktiv gegenüber der Permeabilität aus. Je feinporiger ein Material, desto höher ist die Kapillarkraft, die ein benetzendes Fluid aufzusaugen vermag, desto höher ist aber auch die Reibungskraft, die den Flüssigkeitstransport hemmt. Mit steigender Viskosität der Flüssigkeit nimmt diese Hemmung noch zu. Demgegenüber besitzen grobporige Strukturen eine höhere Permeabilität und ermöglichen auch viskoseren Fluiden einen schnelleren Durchgang.

Im Falle partikelhaltiger Flüssigkeiten tritt ein weiterer hemmender Effekt auf, die mechanische Verblockung. Selbst grobmaschige Strukturen neigen zum Verblocken, wenn die Maschenweite nicht deutlich größer als die Partikeldimension ist. Mit Vliesstoffen sind derart grobe Poren innerhalb der Faserstruktur nicht zu erreichen, wenn gleichzeitig ein hoher Anspruch an Weichheit und Hautfreundlichkeit gestellt wird, der extrem grobe und damit steife Fasern verbietet.

Zu den partikelhaltigen, viskosen Körperflüssigkeiten zählen beispielsweise Menstruationsflüssigkeit, dünne Darmabgänge, Neugeborenenstuhl, Blut und Wundsekret. Diese Flüssigkeiten werden von normalen Vliesstrukturen nicht aufgenommen und transportiert. Es tritt lediglich kapillare Entwässerung ein, die darauf beruht, dass durch den Kapillareffekt Wasser aus den heterogenen Flüssigkeiten entzogen wird, wobei diese selbst eingedickt werden. Zurück bleibt ein noch zähflüssigeres Fluid mit breiartiger Konsistenz, das bei längerem Hautkontakt nicht selten zu Irritationen und entzündlichen Reizungen führt.

Es hat daher nicht an Versuchen gefehlt, diese Mängel durch Aufprägung einer Überstruktur mit Erhöhungen und Vertiefungen bzw. Faltungen zu beheben.

Das Patent WO2014068492 beschreibt beispielsweise ein Verfahren, mit dem ein Vliesstoff mit Hohlnoppenstruktur gebildet wird. Dieser Vliesstoff vermag zumindest in den Vertiefungen eine gewisse Menge an heterogenem, viskosem Fluid aufzunehmen. Das Fluid verbleibt aber auf der Körperseite und wird nicht innerhalb des Vliesstoffverbundes weitergeleitet.

Das Patent DE10296874 beschreibt hingegen ein Verbundmaterial, das aufweist: eine erste Schicht, die eine Vielzahl von Öffnungen enthält; und einen Unteraufbau, der an die erste Schicht angelegt ist, worin der Unteraufbau und die erste Schicht eine Vielzahl von Hohlräumen zum Unterbringen eines Durchgangs von Fluiden durch das Verbundmaterial begrenzen. Nachteilig ist, dass der Verbund aus zwei einzeln hergestellten Lagen besteht, die erst nach den Formgebungsverfahren miteinander verbunden werden. Es ist daher nicht möglich, die Perforationen der Oberlage mit den Hohlräumen der Unterlage in idealer Weise zur Deckung zu bringen. Ohne Deckung wird jedoch der Lochquerschnitt verringert, was die Neigung zur Verblockung stark erhöht.

Aus der DE1013627 sind ADL-Lagen als strukturierte Vliesstoffe bekannt, die eine parallel zueinander verlaufende Struktur aus verschieden stark komprimierten Faserbereichen aufweisen. Obwohl diese Vliesstoffe in den stark komprimierten Bereichen eine gute Flüssigkeitsverteilung gewährleisten, sind so hergestellte Vliesstoffe aufgrund der thermischen Fixierung der stark komprimierten Bereiche eher steif und unflexibel. Des Weiteren ist die Aufnahme von zähflüssigen oder pastösen, partikelhaltigen Körperausscheidungen nur im Bereich der sich zwischen den stark und gering komprimierten Bereichen ergebenden Zwischenräume möglich. Derartige Vliesstoffe benötigen daher immer eine Abdecklage, um ggf die vorbeschriebenen Körperausscheidungen zu überdecken.

Der Einsatz von gelochten Vliesstoffen wiederum löst das Problem des Handlings von pastösen Körperausscheidungen, jedoch sind derartige Vliesstoffe, bedingt durch die Perforation mechanisch nicht hoch belastbar. Des Weiteren benötigen derartige Vliese eine nachgeschaltete ADL-Schicht um die Körperausscheidungen über den Saugkern gleichmäßig zu verteilen. Gattungsbildend ist hier beispielsweise die DE102006033071. Benötigt werden also wiederum zwei Lagen, wobei erst deren Kombination ein sinnvolles Handling von Körperausscheidung ermöglicht.

Aus dem Stand der Technik der EP423619 sind des Weiteren Laminate bekannt, bei welchen Filamentvliesstoffe mit Stapelfaservliesstoffen hydrodynamisch verbunden und strukturiert werden. Die mechanischen Eigenschaften sind bei derartigen Kombinationen zwar verbessert, nachteilig ist aber ebenfalls das nur unzureichende Handling von pastösen Körperausscheidungen.

Die Aufgabe der Erfindung ist es daher, ein Vliesstofflaminat bereitzustellen, welches neben ausreichenden mechanischen Festigkeiten und Weichheit auch in der Lage ist, pastöse Körperausscheidungen aufzunehmen und über zielgerichtet innerhalb eines Hygieneartikels zu verteilen.

Die vorliegende Erfindung löst die Aufgabe, indem sie ein mindestens zweilagiges Vliesstofflaminat V aus einem kardierten Vliesstoff mit einem Filamentvliesstoff bereitstellt.

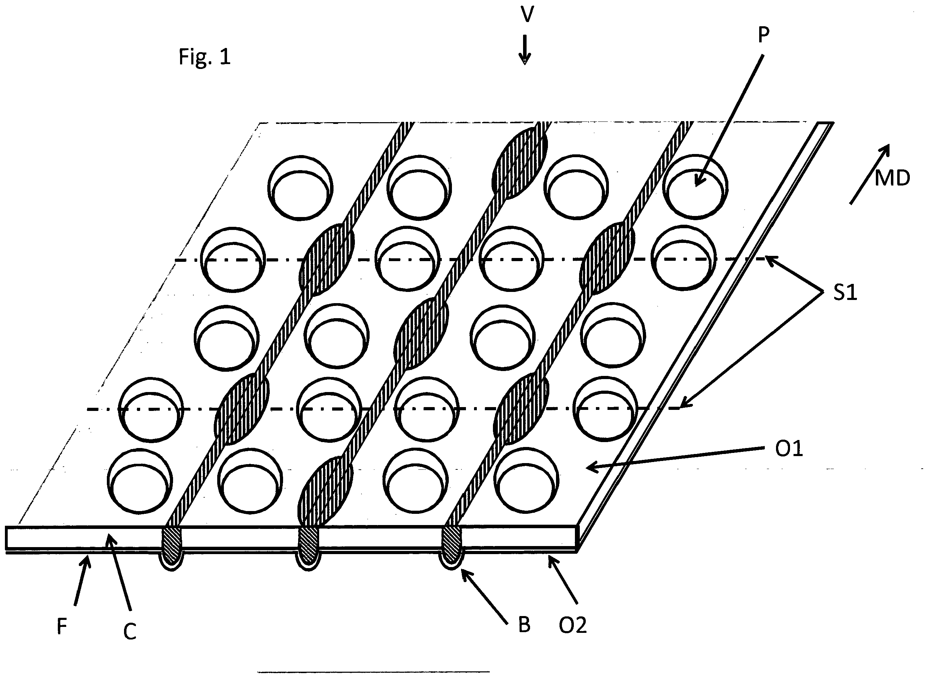

Gemäß der Figur 1 weist das Vliesstofflaminat V dabei zwei unterschiedliche Oberflächen auf:

- Die Oberfläche O1, welche weitestgehend planar ist und regelmäßig angeordnete Perforationen P aufweist, welche das Vliesstofflaminat durchdringen und bis zur Oberfläche 02 reichen.
- Die Oberfläche O2, welche zusätzlich zu den Perforationen P aus der Fläche herausragende, parallel zu einander verlaufende Erhöhungen B aufweist.

Dadurch entstehen auf der Oberfläche 02 Kanäle, die sich entlang der Oberfläche 02 ziehen und seitlich von den Erhöhungen B begrenzt werden.

Setzt man nun ein erfindungsgemäßes Vliesstofflaminat V beispielsweise in einem Hygieneprodukt ein, so kann die Oberfläche 01 zum Benutzer hin angeordnet werden. Die Oberfläche 02 zeigt dann zum Saugkern hin.

Auftreffende Flüssigkeiten dringen durch die Perforationen P in die Struktur ein. Abhängig von der Viskosität werden diese dann mittels zweier Mechanismen entlang der Kanäle auf der Oberfläche 02 verteilt:

a) Niedrigviskose Flüssigkeiten werden aufgrund von Kapillarkräften innerhalb der Erhöhungen B transportiert,
b) Pastöse Flüssigkeiten werden druckgetrieben innerhalb der Kanäle weitergeleitet.

Druckgetrieben bedeutet, dass während der Anwendung bspw in einer Windel durch das Sitzen Druck senkrecht zur möglichen Bewegungsrichtung der Flüssigkeit ausgeübt wird, sodass die Flüssigkeit entlang der Kanäle diesem Druck ausweichen kann.

Im Folgenden werden die Begriffe Stapelfasern, kardierter Vliesstoff, Filamentvliesstoff und Spin-Finish genannt, wobei diese wie folgt definiert sind:

Stapelfaser: bezeichnet eine Faser mit endlicher Länge. Übliche Faserlängen sind im Bereich von 10 - 70mm, bevorzugt 35-50mm. Die Faserfeinheiten liegen im Bereich von 1,0 bis 3,3dtex, bevorzugt 1,3 bis 2,2 dtex. Die Querschnittsform der Stapelfasern können dabei entweder rund / ellipsoid sein, es sind aber auch andere Querschnitte wie bspw. gelappt möglich. Die zum Einsatz kommenden Stapelfasern können Synthesefasern, aber auch Naturfasern sein. Ohne darauf beschränkt zu sein, bestehend Stapelfasern im Sinne dieser Erfindung vorzugsweise aus thermoplastischen Polymeren wie Polyolefinen (PP, PE, PP/PE, CoPP, PA ...), Polyestern (PET, CoPET, PLA, PEF = Polyethylene Furanoate) und aus synthetischen und/oder natürlichen cellulosischen Fasern wie Baumwolle, Viskose oder Lyocell. Die Stapelfasern können dabei sortenrein oder in beliebigen Mischverhältnissen zueinander zum Einsatz kommen.

Der Begriff kardierter Vliesstoff bezeichnet unter Verwendung von Stapelfasern hergestellte Vliesstoffe, welche mittels des Kardierprozesses hergestellt wurden. Das zugrundeliegende Verfahren kann dem im Jahr 2012 bei Wiley VCH-Verlag, Weinheim erschienenen Buch "Vliesstoffe", Seite 136 ff entnommen werden.

Der Begriff Filamentvliesstoff bezeichnet Vliesstoffe, welche nach einem Extrusionsverfahren wie dem Spinnvliesverfahren, dem Meltblown-Verfahren oder der Kombination dieser beiden Verfahren, den sogenannten Spinnvlies / Meltblown / Spinnvlies, kurz S/M/S-Verfahren hergestellt und thermisch mittels Kalandern verfestigt wurden. Erfindungsgemäße Filamentvliese bestehen aus thermoplastischen Polymeren, vorzugsweise aus Polyolefinen (PP, PE, PP/PE, CoPP, PA) oder Polyestern (PET, CoPET, PLA, PEF = Polyethylene Furanoate). Die Filamentfeinheiten bei den Spinnvliesen liegen im Bereich von 1,0 bis 3,3dtex, bevorzugt 1,3 bis 2,2 dtex. Die zugrundeliegenden Herstellungsverfahren können dem im Jahr 2012 bei Wiley VCH-Verlag, Weinheim erschienenen Buch "Vliesstoffe", Seite 175 ff entnommen werden.

Spin-Finish bezeichnet eine funktionelle Beschichtung von Faser- oder Filamentoberflächen. Das Ziel ist dabei entweder die Verarbeitungseigenschaften oder die Gebrauchseigenschaften zu beeinflussen. Beispiele dafür sind Antistatika oder Hydrophilierungs- oder Hydrophobierungsmittel. Eine grundsätzliche Beschreibung kann dem im Jahr 2012 bei Wiley VCH-Verlag, Weinheim erschienenen Buch "Vliesstoffe", Seite 83 ff entnommen werden.

Der Begriff pastös steht dabei für Flüssigkeiten deren Viskosität im Bereich von 4 bis 150 mPas liegen (gemessen mit einem Rotationsviskosimeter bei 20°C).

Die dabei ermittelten Parameter wurden gemäß den nachfolgend genannten Prüfmethoden ermittelt:

- Flächengewicht gemäß WSP 130.1., Angabe in g/m$^2$
- Steighöhe gemäß WSP 010.1., Absatz 7.3, Angabe in mm
- Dicke gemäß WSP 120.6, Messdruck 0,5kPa, Angabe in mm
- Strike Through gemäß WSP 70.7, Angabe in s
- Rewet gemäß WSP 70.8, Angabe in g
- Run off in Anlehnung an WSP 80.9, abweichend zu dieser Norm wird mit 75 ml statt 50 ml NaCI-Lösung gearbeitet, die schiefe Ebene hat ein Gefälle von 25° statt 45°. Die Berechnung des RunOff erfolgt dabei nach nachfolgender Formel:

$$\text{Run off } [\%] = \frac{Run\ off\ Menge\ in\ g}{Aufgebrachte\ Flüssigkeitsmenge\ in\ g} * 100$$

Zur Herstellung des erfindungsgemäßen Vliesstofflaminats V wird ein kardiertes Vlies C aus Stapelfasern auf ein vorverfestigtes Filamentvlies F, beispielsweise ein Spinnvlies, abgelegt und mittels Wasserstrahlvernadelung miteinander verbunden.

Der so entstandene, noch unstrukturierte Vorverbund wird anschließend einer Strukturtrommel zugeführt, um so die erfindungsgemäße Struktur durch eine weitere Wasserstrahlbehandlung des Vorverbunds herzustellen

Diese Strukturtrommel weist nun auf der Trommeloberfläche mustermäßig verteilte Öffnungen L und aus der Oberfläche herausragende Perforationselemente K auf.

Durch die Festlegung der Abstände der Öffnungen L zueinander und der Anordnung der Perforationselemente P um die Öffnungen L wird während des Strukturierungsvorganges eine Akkumulation von durch die Wasserstrahleinwirkung verdrängten Fasern und Filamente derart bewirkt, dass sich die Erhöhungen B bilden.

Bevorzugt und ohne darauf beschränkt zu sein wird dabei die Anordnung der Perforationselemente K in einem hexagonalen Muster, wobei die Perforationselemente K jeweils die Eckpunkte der Hexagons besetzen. In der Mitte eines jeweiligen von den Perforationselementen begrenzten Hexagons ist eine Öffnung L angeordnet.

Die Ausrichtung der Hexagons auf der Oberfläche der Strukturtrommel ist dabei durch die Symmetrieachse S1 bestimmt. Die Symmetrieachse S1 beschreibt die Symmetrielinie, welche durch zwei gegenüberliegende Hexagon-Ecken verläuft.

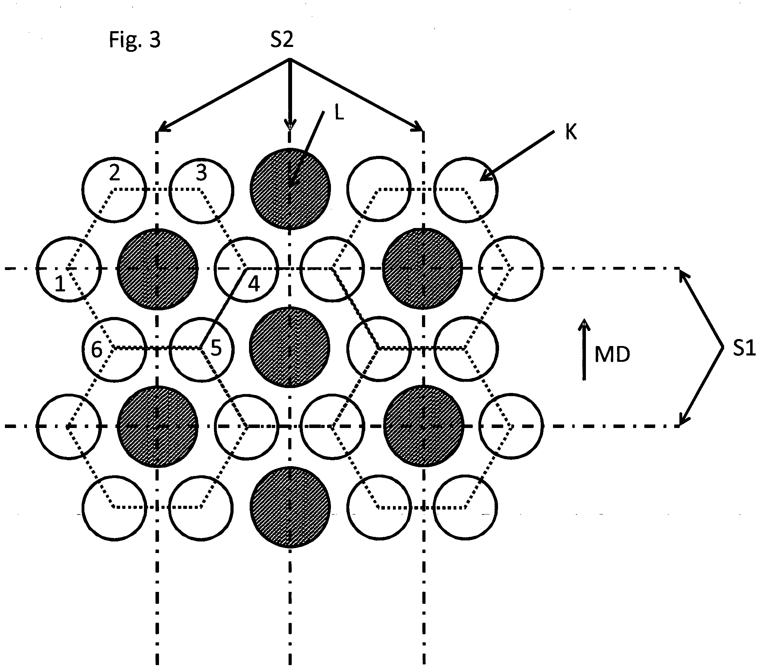

Zur Verdeutlichung dient die Figur 3. Sie zeigt schematisch die räumliche Anordnung der Perforationselemente K, beispielsweise in Form von Kegeln, auf der Oberfläche der Strukturtrommel. Zur besseren Erkennbarkeit der hexagonalen Struktur wurden die Kegelspitzen mit gepunkteten Hilfslinien verbunden.

Ebenfalls zur Kennzeichnung wurden die Kegelspitzen eines Hexagons mit Nummern versehen. Die Symmetrielinie S 1 ergibt sich durch die Verbindung der Kegelspitzen 1 und 4. Aus Figur 3 ist auch die Lage der Öffnungen L entlang der Symmetrielinie S2 ersichtlich. Diese verbindet die Mittelpunkte der Öffnungen L und ist um 90° versetzt zur Symmetrielinie S1.

Die Symmetrielinie S1 kann wie in Figur 3 dargestellt um 90° versetzt, d.h. quer zur Fertigungsrichtung MD verlaufen, sie kann aber auch in einem beliebigen Winkel zwischen 0 und 90° bezogen auf die Fertigungsrichtung MD gewählt werden. Die letztendliche Ausrichtung ist vom späteren Verwendungszweck und der gewünschten Verteilrichtung für Flüssigkeiten in einem Hygieneprodukt abhängig.

Bei der Herstellung des erfindungsgemäßen Vliesstofflaminats V ist zu beachten, dass die Seite des Vorverbunds auf welcher der Filamentvliesstoff angeordnet ist, auf der Strukturtrommel zu liegen kommt. Diese Seite des Vorverbunds bildet im späteren Vliesstofflaminat die Seite 02. Die Seite, auf welcher das kardierte Vlies liegt, ist im späteren Vliesstofflaminat die Seite O1.

Während des Strukturierungsvorganges wird der Vorverbund durch die auftreffenden Wasserstrahlen auf die Perforationselemente K gedrückt. Durch den dabei herrschenden Wasserdruck dringen die Perforationselemente K in den Vorverbund ein, sodass der Vorverbund, insbesondere das Filamentvlies an diesen Stellen aufreißt und dadurch die Perforationen P im erfindungsgemäßen Vliesstoffverbund V gebildet werden. Die Fasern / Filamente des Vorverbunds werden dabei seitlich entlang der Flanken der Perforationselemente K verdrängt.

Überraschenderweise wurde festgestellt, dass sich durch die erfindungsgemäß gewählte Anordnung der Perforationselemente K in Verbindung mit den Öffnungen L die verdrängten Fasern sich entlang der Symmetrieachse S2 akkumulieren und durch die Wasserstrahlbehandlung miteinander verwirbelt werden. Diese aus verdrängten Fasern bestehenden Verwirbelungen bilden die erfindungsgemäß beanspruchten Erhöhungen B, welche kontinuierlich entlang der Symmetrielinie S2 laufen.

Erklärt wird dies aus dem Zusammenspiel der erfindungsgemäßen Ausrichtung der Peforationselemente K auf der Strukturtrommel in Verbindung mit den Öffnungen L.

Während des Perforationsvorganges akkumulieren sich die von den Perforationselementen K verdrängten Fasern und Filamente vornehmlich entlang der Symmetrielinie S2 und werden durch den herrschenden Wasserdruck in die Öffnungen L gedrückt. Die im kardierten Vliesstoff C und dem Filamentvliesstoff F vorhandenen Filamente und Fasern überbrücken dabei die Distanz zwischen zwei Öffnungen L entlang der Symmetrieachse S2, sodass sich die Verwirbelungen und in der Folge die erfindungsgemäßen Erhöhungen B kontinuierlich entlang der Symmetrieachse S2 ergeben. Ein Teil der Faser des kardierten Vliesstoffes C wird dabei durch den Filamentvliesstoff gedrückt.

Wie der Figur 1 schematisch zu entnehmen, variiert die Breite der Erhöhungen B dabei. Im Bereich der Mitte eines Hexagons sind die Erhöhungen B breiter als im Bereich zwischen Perforationen P.

Nach der so erfolgten Strukturbehandlung erhält man das erfindungsgemäße Vliesstofflaminat V, welches eine plane, perforierte Oberfläche O1 und eine perforierte und mit Erhöhungen B versehene Oberfläche 02 aufweist.

Die Stapelfaserlage auf der Seite O1 bildet dabei eine überwiegend glatte Seite mit angenehmer Weichheit und hohem Tragekomfort, während die Seite 02 infolge der Faserverdichtung um die Perforationen herum in die Hohlräume der Strukturtrommel gedrückt wird und dabei die linienförmigen Erhöhungen B bildet. Ein wesentlicher Aspekt dabei ist die Verdichtung der Fasern um die Löcher P herum. Durch diese Verdichtung werden die Erhöhungen B stabilisiert und bleiben auch unter Druck erhalten. Die Erhöhungen B bilden einerseits dazwischenliegende Hohlräume in Form von Kanälen, die hochpermeabel für Flüssigkeiten sind, insbesondere für viskose und partikelhaltige, andererseits bilden die verdichteten Fasern innerhalb der Erhöhungen B parallele Stränge mit hoher Kapillarität, welche auch niedrigviskose Flüssigkeiten aktiv aufzusaugen vermögen.

Eine bevorzugte Ausführungsform der Erfindung sieht vor, dass die Symmetrieachse S1 quer zur Fertigungsrichtung MD verläuft. In der späteren Anwendung in einem Hygieneprodukt wird durch die von der Stapelfaserlage gebildete Seite 01 dabei als Toplage hautseitig orientiert, während die Filamentlage auf der Seite 02 mit der von ihr umschlossenen Kanalstrukturen zum Absorberkern hin orientiert ist. Da die Perforationen P durchgängig und direkt in die Kanalstrukturen münden, ist die Flüssigkeitsaufnahme gegenüber dem Stand der Technik wesentlich verbessert.

Die Ausrichtung der Kanäle und Erhöhungen B in Maschinenrichtung ermöglicht einen gerichteten Flüssigkeitstransport, der innerhalb des Hygieneproduktes für eine optimale Flüssigkeitsverteilung sorgt. Dabei kommen gleichzeitig die Kapillarität der Faserstränge innerhalb der Erhöhungen B und die hohe Permeabilität der makroskopischen Kanäle zur Wirkung.

Die Körperfluide werden entlang der Kanäle direkt zum Absorberkern geleitet. Dieser vermag die Fluide zwar nicht als Gesamtheit zu absorbieren, die polymeren Absorber vermögen aber aufgrund ihrer gelartigen Struktur die Körperfluide viel stärker kapillar zu entwässern, als Faservliese dazu in der Lage wären. Dies führt zu einer weitgehenden Entwässerung, Volumenverringerung und Immobilisierung der Fluidrückstände in den Hohlraumstrukturen des Verbundmaterials.

Da Kapillarität an Feinporigkeit geknüpft ist, vermögen polymere Superabsorber mit ihren Poren molekularer Dimension um Größenordnungen stärkere Kapillarkräfte zu entfalten als feinstporige Faservliese. Die Entwässerung der heterogenen Fluide, die in eingedickter Form selbst gelartiger Natur sind, gelingt somit in stärkerem Maße als wenn die Fluide nur auf der Decklage mit Fasern in Kontakt kommen und einen zu großen Abstand zum Absorberkern haben.

In einer beispielhaften Ausführung des erfindungsgemäßen Vliesstofflaminats wurde ein Flor aus Polyethylenterephthalat-Stapelfasern in einem Titer von 1,3 dtex und einer Stapellänge von 38mm und einem Florgewicht von 35g/m$^2$ auf einem Filamentvliesstoff vom Typ Spinnvlies aus Polypropylenfilamenten mit einem Flächengewicht von 10 g/m$^2$ abgelegt.

Die Lagen des so entstandenen Aufbaus werden mittels Wasserstrahlvernadelung bei einem mittleren Druck von 90 bar mit einander verbunden, sodass der Vorverbund entsteht.

Anschließend wird der Vorverbund einer Strukturiereinheit, bestehend aus einer Strukturtrommel und zwei mit Lochstreifen versehenen Balken zur Erzeugung der Wasserstrahlen zugeführt. An den Wasserstrahlbalken liegt ein durch Hochdruckpumpen erzeugter Druck von 180 bar an.

Die Strukturtrommel hat eine erfindungsgemäß ausgeführte Oberfläche, bei welcher ausgehend von der Figur 3

- die Perforationselemente aus Kegeln bestehen, deren Kegelfuß auf der Strukturtrommel einen Durchmesser von 1,8mm aufweist,

- die Perforationselemente in Form eines regulären Hexagons mit einer Kantenlänge von 3,8mm angeordnet sind

- die Öffnungen L mittig innerhalb der Hexagons angeordnet sind

- die Öffnungen L als Kreise mit einem Durchmesser von 4,4mm ausgeführt sind.

Nach erfolgter Strukturierung wurde einseitig auf die Oberfläche 02 ein hydrophiler Spin-Finish aufgebracht, sodass der Vliesstoffverbund V zwei unterschiedlich hydrophile Seiten aufweist. Die Oberfläche 01 ist weniger hydrophil als die Seite 02, sodass auf der Seite O1 auftreffende Flüssigkeiten durch die Perforationen schnell auf die Seite 02 weitergeleitet werden.

Das so strukturierte Vliesstofflaminat hat nach Abschluss der Behandlung folgende Eigenschaften:

Tabelle 1:

| Lochdurchmesser (Lineal) | [mm] | 1,7 |
|---|---|---|
| Abstand der Erhöhungen B (Lineal, gemessen entlang der Symmetrielinie S2) | [mm] | 4,8 |
| Flächengewicht | [g/m$^2$] | 45 |
| Dicke | [mm] | 1 |
| Strike Through | [s] | 2,0 |
| Rewet | [g] | 0,08 |
| Steighöhe (nach 120 s) | [mm] | 30 |
| Run-Off (Neigungswinkel 25°) | [%] | 0 |

Neben den im Ausführungsbeispiel genannten Angaben können die Strukturen eines erfindungsgemäß ausgeführten Vliesstofflaminats V auch durch die Form der Perforationselemente P beeinflusst werden. Die Perforationselemente P müssen lediglich in der Lage sein, den Vorverbund an den definierten Stellen aufzureißen um eine anwendungsbezogene Lochfläche von 0,5 mm$^2$ bis 20 mm$^2$, bevorzugt von 3 mm$^2$ bis 10 mm$^2$ zu erzeugen.

Geringe Lochflächen bis 1mm$^2$ sind hauptsächlich für das Management niedrigviskoser Flüssigkeiten geeignet, Lochflächen ab 3mm$^2$ eignen sich für höherviskose Flüssigkeiten. Begrenzt nach oben wird die Lochfläche durch die Stabilität des Vliesstofflaminats. Lochflächen von größer 20mm$^2$ führen zu einem Vliesstofflaminat was nur unzureichende mechanische Festigkeiten aufweist.

Die Abstände der Erhöhungen B zueinander können in einem Bereich von 2 mm bis 15 mm, besonders bevorzugt 4 mm bis 7 mm zueinander gewählt werden. Die Wahl richtet sich auch hier nach dem Einsatzzweck. Geringe Abstände zueinander bewirken eine höhere Rückstellkraft im Verbund, größere Abstände zueinander bewirken ein weicheres Vlies.

Die Ausprägung der Erhöhungen B wird des Weiteren von den Flächengewichten der eingesetzten kardierten Vliesstoffen und / oder den Filamentvliesstoffen beeinflusst. Je höher die Flächengewichte liegen, umso mehr Fasermasse kann beim Strukturieren verdrängt werden, entsprechend stärker fällt die Ausprägung der Erhöhungen B aus. Erfindungsgemäße Flächengewichtsbereiche liegen beim kardierten Vliesstoff C im 5 g/m$^2$ bis 80 g/m$^2$ und bevorzugt von 20 g/m$^2$ bis 40 g/m$^2$. Für den Filamentvliesstoff ist ein Flächengewicht von von 5 g/m$^2$ bis 40 g/m$^2$, bevorzugt von 10 g/m$^2$ bis 20 g/m$^2$ einsetzbar.

Auch die Art und der Anteil von Spinfinish auf dem Vliesstofflaminat V ist anwendungsabhängig zu sehen. Durch den Wasserstrahlprozess wird der auf den Fasern als Verarbeitungshilfe vorhandene Spinfinish zu großen Teilen abgewaschen, sodass der Vliesstoffverbund V zunächst hydrophob vorliegt.

Um nun die gewünschte Hydrophilie zu bekommen, muss das Vliesstofflaminat nachträglich mit einem hydrophilen Spinfinish behandelt werden. Üblich sind dabei auflagen von 0,05 bis 1,0 Massenprozent, wobei ein Bereich von 0,2 bis 0,8 Massenprozent bevorzugt wird.

Der Auftrag des Spinfinish kann dabei einseitig, bevorzugt auf die Oberfläche 02 des erfindungsgemäß ausgeführten Vliesstofflaminats, stattfinden. Es ist aber auch ein beidseitiger Auftrag möglich.

Bevorzugt werden Spinfinish-Auflagen die auf Oberfläche 02 stärker hydrophile Eigenschaften bewirken als auf der Oberfläche O1, so dass sich ein Gradient zwischen beiden Oberflächen einstellt.

Erfindungsgemäß ausgeführte Vliesstofflaminate können neben dem Einsatz in persönlichen Hygieneprodukten auch in anderen Bereichen wie beispielsweise Reinigungstüchern, Desinfektionstüchern Einsatz finden.

Bezugszeichenliste:

| | |
|---|---|
| V | = Vliesstofflaminat |
| P | = Perforation im Vliesstofflaminat |
| O1 | = Unstrukturierte Oberfläche des Vliesstofflaminats |
| O2 | = Strukturierte Oberfläche des Vliesstofflaminates |
| B | = Erhöhung im Vliesstofflaminat |
| C | = kardierter Vliesstoff |
| F | = Filamentvliesstoff |
| S1 | = Symmetrieachse 1 |
| S2 | = Symmetrieachse 2 |
| K | = Perforationselemente auf der Strukturschale |
| L | = Öffnungen in der Strukturschale |
| MD | = Fertigungsrichtung |
| 1 - 6 | = Kegelspitzen |

**Patentansprüche**

1. Vliesstofflaminat, bestehend aus einem kardierten Stapelfaservliesstoff und einem Filamentvliesstoff, welche mechanisch mittels hydraulischer Vernadelung verbunden sind,
**dadurch gekennzeichnet, dass**

- der Oberflächen O1 und O2 des Vliesstofflaminats mustermäßig verteilte Perforationen P aufweisen,
- der Oberfläche 02 des Vliesstofflaminates eine Struktur aus parallel zueinander verlaufenden Erhöhungen B aufweist und
- dass die Perforationen P zwischen den Erhöhungen B liegen.

2. Vliesstofflaminat nach Anspruch 1,
**dadurch gekennzeichnet, dass**

- die Perforationen P in einem hexagonalem Muster angeordnet sind und dass
- die Erhöhungen B in einem Winkel von 90° zu der die Ausrichtung des hexagonalen Musters der Perforationen P bestimmenden Symmetrieachse S1 verlaufen.

3. Vliesstofflaminat nach Anspruch 1 und 2,
**dadurch gekennzeichnet, dass**

- die Oberfläche 01 von dem kardierten Vliesstoff gebildet wird
- die Oberfläche 02 vom Filamentvliesstoff gebildet wird.

4. Vliesstofflaminat nach einem der vorhergehenden Ansprüche,
**dadurch gekennzeichnet,**
**dass** die Perforationen P kreisförmige und/oder ellipsoide und/oder irreguläre Gestalt besitzen und eine Lochfläche von 0,5 mm$^2$ bis 20 mm$^2$, bevorzugt von 3mm$^2$ bis 10 mm$^2$ aufweisen.

5. Vliesstofflaminat nach einem der vorhergehenden Ansprüche,
**dadurch gekennzeichnet,**
**dass** die Erhöhungen B vorzugsweise in MD orientiert sind und im Abstand von 2 mm bis 15 mm, besonders bevorzugt 4 mm bis 7 mm zueinander verlaufen.

6. Vliesstofflaminat nach einem der vorhergehenden Ansprüche,
**dadurch gekennzeichnet,**
**dass** die Oberfläche 02 hydrophile Eigenschaften besitzt und die Oberfläche O1 weniger hydrophile bis hydrophobe Eigenschaften aufweist, so dass zumindest ein Gradient zwischen beiden Oberflächen besteht.

7. Vliesstofflaminat nach einem der vorhergehenden Ansprüche,
   **dadurch gekennzeichnet,**
   **dass** der Filamentvliesstoff ein Spinnvliesstoff oder eine Kombination aus Spinnvliesstoffen mit Extrusionsvliesstoffen darstellt.

8. Vliesstofflaminat nach einem der vorhergehenden Ansprüche
   **dadurch gekennzeichnet,**
   **dass** die kardierte Stapelfaservlieslage ein Flächengewicht nach DIN EN 29073-1 von 5 g/m$^2$ bis 80 g/m$^2$, vorzugsweise von 10 g/m$^2$ bis 60 g/m$^2$ und besonders bevorzugt von 20 g/m$^2$ bis 40 g/m$^2$ aufweist und der Filamentvliesstoff ein Flächengewicht von von 5 g/m$^2$ bis 40 g/m$^2$, vorzugsweise von 8 g/m$^2$ bis 25 g/m$^2$ und besonders bevorzugt von 10 g/m$^2$ bis 20 g/m$^2$ aufweist.

9. Vliesstofflaminat nach einem der vorhergehenden Ansprüche
   **dadurch gekennzeichnet,**
   **dass** die Stapelfasern vorzugsweise aus thermoplastischen Polymeren wie Polyolefinen (PP, PE, PP/PE, CoPP, PA ...) und/oder Polyestern (PET, CoPET, PLA, PEF = Polyethylene Furanoate, ...) und zumindest anteilig aus synthetischen und/oder natürlichen cellulosischen Fasern (CV, CO, ...) bestehen und das Filamentvlies ebenfalls aus thermoplastischen Polymeren, vorzugsweise aus PP- oder CoPP- oder PP/PE- oder PET/PE besteht.

Fig. 1

V

P

MD

S1

O1

O2

B

F  C

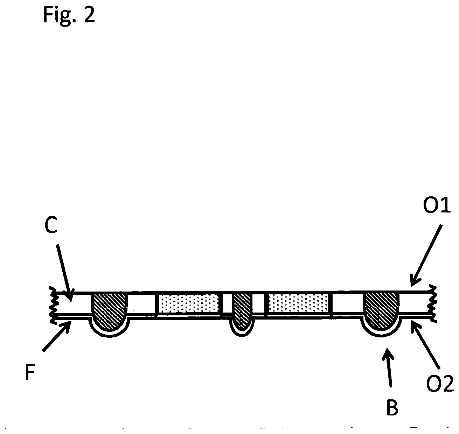

Fig. 2

Fig. 3

Europäisches
Patentamt

European
Patent Office

Office européen
des brevets

**EUROPÄISCHER RECHERCHENBERICHT**

Nummer der Anmeldung

EP 17 00 2000

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | US 2014/121621 A1 (KIRBY SCOTT S C [AU] ET AL) 1. Mai 2014 (2014-05-01) * Absatz [0144] - Absatz [0150] * * Absatz [0157] - Absatz [0160] * * Absatz [0168] - Absatz [0169]; Abbildungen 7-9 * ----- | 1-9 | INV. A61F13/511 A61F13/512 B32B5/02 B32B5/26 D04H1/495 D04H1/498 D04H13/00 |
| X | WO 2016/040618 A2 (PROCTER & GAMBLE [US]) 17. März 2016 (2016-03-17) * Seite 15, Zeile 4 - Seite 19, Zeile 12 * * Seite 21, Zeile 23 - Seite 26, Zeile 11 * * Seite 27, Zeile 13 - Zeile 32; Abbildungen 7-10 * ----- | 1-9 | |
| Y | EP 2 034 072 A1 (UNI CHARM CORP [JP]) 11. März 2009 (2009-03-11) * Absatz [0112] - Absatz [0137] * * Absatz [0148] - Absatz [0150] * * Absatz [0189] - Absatz [0190]; Ansprüche 1, 6, 8, 13, 19, 21; Abbildungen 12, 13A, 13B, 21, 22 * ----- | 1-9 | |
| Y | DE 10 2013 111499 A1 (ASCANIA NONWOVEN GERMANY GMBH [DE]) 23. April 2015 (2015-04-23) * Absatz [0021] - Absatz [0024] * * Absatz [0068] - Absatz [0080]; Ansprüche 1, 6, 13, 14; Abbildungen 1, 4, 6 * ----- | 1-9 | RECHERCHIERTE SACHGEBIETE (IPC) A61F B32B D04H |
| A,P | WO 2017/156200 A1 (PROCTER & GAMBLE [US]) 14. September 2017 (2017-09-14) * Seite 31 - Seite 32 * * Seite 41, Zeile 30 - Seite 43, Zeile 10; Ansprüche 1, 3, 9, 11-13; Abbildung 28 * ----- | 1-9 | |

Der vorliegende Recherchenbericht wurde für alle Patentansprüche erstellt

| Recherchenort | Abschlußdatum der Recherche | Prüfer |
|---|---|---|
| München | 12. Februar 2018 | Demay, Stéphane |

KATEGORIE DER GENANNTEN DOKUMENTE

X : von besonderer Bedeutung allein betrachtet
Y : von besonderer Bedeutung in Verbindung mit einer
 anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
 nach dem Anmeldedatum veröffentlicht worden ist
D : in der Anmeldung angeführtes Dokument
L : aus anderen Gründen angeführtes Dokument

..............................................................................................
& : Mitglied der gleichen Patentfamilie, übereinstimmendes
 Dokument

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 17 00 2000

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

12-02-2018

| Im Recherchenbericht angeführtes Patentdokument | Datum der Veröffentlichung | Mitglied(er) der Patentfamilie | Datum der Veröffentlichung |
|---|---|---|---|
| US 2014121621 A1 | 01-05-2014 | AU 2013340403 A1 | 11-06-2015 |
| | | CN 104994821 A | 21-10-2015 |
| | | KR 20150081307 A | 13-07-2015 |
| | | NZ 708479 A | 31-03-2017 |
| | | RU 2015119770 A | 20-12-2016 |
| | | US 2014121621 A1 | 01-05-2014 |
| | | WO 2014068488 A1 | 08-05-2014 |
| WO 2016040618 A2 | 17-03-2016 | CA 2959817 A1 | 17-03-2016 |
| | | CN 106604710 A | 26-04-2017 |
| | | EP 3193801 A2 | 26-07-2017 |
| | | JP 2017528612 A | 28-09-2017 |
| | | US 2016067118 A1 | 10-03-2016 |
| | | WO 2016040618 A2 | 17-03-2016 |
| EP 2034072 A1 | 11-03-2009 | EP 2034072 A1 | 11-03-2009 |
| | | JP 5328089 B2 | 30-10-2013 |
| | | JP 2008025081 A | 07-02-2008 |
| | | KR 20090023341 A | 04-03-2009 |
| | | MY 151023 A | 31-03-2014 |
| | | US 2008085399 A1 | 10-04-2008 |
| | | US 2009282660 A1 | 19-11-2009 |
| | | WO 2007148533 A1 | 27-12-2007 |
| DE 102013111499 A1 | 23-04-2015 | CN 105934546 A | 07-09-2016 |
| | | DE 102013111499 A1 | 23-04-2015 |
| | | DE 112014004763 A5 | 11-08-2016 |
| | | EP 3058127 A1 | 24-08-2016 |
| | | JP 2016537525 A | 01-12-2016 |
| | | US 2016243788 A1 | 25-08-2016 |
| | | WO 2015055177 A1 | 23-04-2015 |
| WO 2017156200 A1 | 14-09-2017 | US 2017258645 A1 | 14-09-2017 |
| | | US 2017258648 A1 | 14-09-2017 |
| | | US 2017258649 A1 | 14-09-2017 |
| | | WO 2017156196 A1 | 14-09-2017 |
| | | WO 2017156197 A1 | 14-09-2017 |
| | | WO 2017156200 A1 | 14-09-2017 |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2014068492 A
- DE 10296874
- DE 1013627
- DE 102006033071
- EP 423619 A

**In der Beschreibung aufgeführte Nicht-Patentliteratur**

- Weinheim erschienenen Buch "Vliesstoffe. Wiley VCH-Verlag, 2012, 136 ff
- Weinheim erschienenen Buch "Vliesstoffe. Wiley VCH-Verlag, 2012, 175 ff
- Weinheim erschienenen Buch "Vliesstoffe. Wiley VCH-Verlag, 2012, 83 ff